# EUROPEAN PATENT SPECIFICATION

(11) **EP 3 875 027 B1**
(45) Date of publication and mention of the grant of the patent: **05.10.2022**
(21) Application number: 21160784.1
(22) Date of filing: 04.03.2021
(51) Int. Cl.: A61B 5/15, A61B 5/151

(54) **BLOOD LANCET**
BLUTLANZETTE
LANCETTE POUR PRISE D'ÉCHANTILLONS SANGUINS

(30) Priority: 04.03.2020 CN 202010142604
(43) Date of publication of application: 08.09.2021
(73) Proprietor: Tianjin Huahong Technology Co., Ltd., Tianjin (CN)
(72) Inventor: ZHANG, Libo, TIANJIN (CN); CUI, Chengzhe, TIANJIN (CN)
(74) Representative: Cabinet Beau de Loménie

(56) References cited:
- EP-A1- 2 399 520
- WO-A1-2011/149455
- US-A1- 2017 172 481

## Description

### TECHNICAL FIELD

The embodiments of the present disclosure relate to the field of medical equipment, in particular to a blood lancet.

### BACKGROUND

In the solution adopted by an existing blood lancet, a lancet core is generally launched by pressing an inner sleeve which is in direct contact with the skin of a patient. However, under the condition that the pressing force of the patient is too large, the situation that the puncture depth of the blood lancet is too deep can occur. In addition, since the inner sleeve is pressed in the mode of integrally pressing the blood lancet, the pressing operation of the inner sleeve is not convenient. In addition, it is necessary to enhance the function of preventing the secondary use of the blood lancet.

### SUMMARY OF THE INVENTION

In order to alleviate or solve at least one aspect of the above-mentioned problems, the present disclosure proposes a blood lancet that is pressed on the top to trigger a blood collection operation.

According to an embodiment of the present disclosure, a blood lancet of the present disclosure includes:
an outer sleeve, provided with a lancet outlet at one end and an accommodating port at the other end, wherein a lancet core guide portion is arranged in the outer sleeve;
an inner sleeve, wherein one end of the inner sleeve enters the outer sleeve from the accommodating port, and the other end of the inner sleeve is located at the outside of the accommodating port;
a lancet core, provided with a lancet body, wherein the lancet core is arranged at one end of the inner sleeve so as to rotate along the circumferential direction with the inner sleeve; and
a spring, arranged between the lancet core and the inner sleeve, and provides power for the lancet core to move axially along the lancet core guide portion toward the lancet outlet,
wherein:
   based on the axial movement of the inner sleeve in the direction toward the lancet outlet, the inner sleeve rotates relative to the outer sleeve from a first circumferential position to a second circumferential position;
   at the first circumferential position, the outer sleeve abuts against the lancet core to prevent the lancet core from launching, and at the second circumferential position, the lancet core is suitable for entering the lancet core guide portion for launching; and
   a first protrusion is arranged on the radial outer side of a cylinder body of the inner sleeve, a first blocking portion is arranged in the outer sleeve, when the inner sleeve is at the first circumferential position, the first protrusion is located on one side of the first blocking portion in the circumferential direction; and when the inner sleeve is at the second circumferential position, the first protrusion is located on the other side of the first blocking portion in the circumferential direction, and the first blocking portion prevents the first protrusion located on the other side of the first blocking portion from moving circumferentially to the one side of the first blocking portion.

Optionally, the outer sleeve is provided with a second blocking portion and a third blocking portion, the second blocking portion and the third blocking portion are arranged in sequence along the circumferential direction, and the third blocking portion is a circumferential inclined extension blocking portion; the other end of the inner sleeve is provided with a second protrusion protruding radially outward, and both the second blocking portion and the third blocking portion are suitable for cooperating with the second protrusion to prevent the second protrusion from moving in the direction from the lancet outlet to the accommodating port; and when the inner sleeve is at the first circumferential position, the second protrusion abuts against the second blocking portion, and when the inner sleeve is at the second circumferential position, the second protrusion abuts against the third blocking portion.

Optionally, the second blocking portion includes a circumferential protrusion arranged on the inner wall of the outer sleeve, the circumferential protrusion protrudes radially inward, and the circumferential protrusion has a blocking surface that is suitable for cooperating with the second protrusion to prevent the second protrusion from moving in the direction from the lancet outlet to the accommodating port.

Optionally, the third blocking portion includes a circumferential inclined extension blocking surface, an axial extension blocking surface is arranged between the circumferential inclined extension blocking surface and the blocking surface, one end of the axial extension blocking surface is connected to the circumferential inclined extension blocking surface, and the other end thereof is connected to the blocking surface.

Optionally, the other end of the inner sleeve is provided with an elastic arm, and the end part of the elastic arm is provided with the second protrusion protruding radially outward from the elastic arm.

Optionally, a cooperation guide inclined surface is arranged in the outer sleeve, and the second protrusion is suitable for abutting against the cooperation guide inclined surface to drive the elastic arm to deform radially inward.

Optionally, the second protrusion is provided with a second convex inclined surface that cooperates with the cooperation guide inclined surface.

Optionally, the second blocking portion includes a groove or a slot formed in the inner wall of the outer sleeve or a through hole passing through the cylinder wall of the outer sleeve, and the groove or the slot or the through hole is suitable for cooperating with the second protrusion to prevent the second protrusion from moving in the direction from the accommodating port to the lancet outlet; or the second blocking portion includes an inclined cantilever, which is elastically deformable radially outward, and one end of which is connected to the inner wall of the outer sleeve while the other end of which extends obliquely inward toward the lancet outlet, the second protrusion extrudes the inclined cantilever radially outward during passing through the inclined cantilever, and after the second protrusion passes through the inclined cantilever, the other end of the inclined cantilever moves radially inward to prevent the second protrusion from moving in the direction from the accommodating port to the lancet outlet.

Optionally, a positioning surface adjacent to the lancet core guide portion is arranged in the outer sleeve, and the lancet core has an abutting end face that abuts against the positioning surface; a rotation guide portion is arranged in the outer sleeve, and one end of the inner sleeve is provided with a rotation cooperation portion that cooperates with the rotation guide portion; and based on the axial movement of the inner sleeve in the direction toward the lancet outlet, the rotation cooperation portion cooperates with the rotation guide portion to cause the inner sleeve to rotate in the circumferential direction, and based on the rotation, the abutting end face is suitable for leaving the positioning surface so as to enter the lancet core guide portion.

Optionally, the inner sleeve is provided with a guide groove, the lancet core is provided with a guide member that cooperates with the guide groove, the guide member is arranged in the guide groove, and the guide groove is suitable for driving the guide member so as to drive the lancet core to rotate along the circumferential direction.

Optionally, one end face of the guide member constitutes the abutting end face.

Optionally, the rotation guide portion includes a circumferential inclined surface; the end part of the guide groove constitutes the rotation cooperation portion to be suitable for being engaged with the circumferential inclined surface; or the outer side of the inner sleeve is provided with a cooperation flange protruding radially outward, and the cooperation flange constitutes the rotation cooperation portion to be suitable for being engaged with the circumferential inclined surface.

Optionally, the rotation guide portion includes an inclined rib arranged in the outer sleeve, and the inclined rib protrudes radially inward and has the circumferential inclined surface.

Optionally, the circumferential inclined surface is located on the outer side of the lancet core guide portion in the radial direction; the outer sleeve is further provided with a circumferential plane that is in contact with the circumferential inclined surface, and in the case that the rotation cooperation portion moves from the circumferential inclined surface to the circumferential plane based on the rotation of the inner sleeve, the guide groove and the lancet core guide portion are aligned in the axial direction to allow the guide member to move from the guide groove to the lancet core guide portion.

Optionally, the rotation guide portion comprises an inclined guide groove; the rotation cooperation portion comprises a cooperation protrusion that protrudes radially outward from the inner sleeve, and the cooperation protrusion is suitable for moving in the inclined guide groove.

Optionally, in the axial direction, an abutting member in the inner sleeve abuts against the lancet core to apply an abutting force toward the direction of the lancet outlet, a component in the outer sleeve abuts against the lancet core to apply an abutting force toward the accommodating port, and the lancet core abuts against the abutting member in the inner sleeve and abuts against the outer sleeve at the same time to prevent the inner sleeve from moving toward the lancet outlet in the axial direction.

Optionally, the lancet core is provided with a supporting surface at the end where the spring is provided, and the abutting member and the supporting surface are suitable for abutting against each other; the lancet core further includes a lancet cap arranged on the lancet body, the lancet cap passes through the outer sleeve through the lancet outlet, the lancet cap is provided with a lancet cap hanging platform, the lancet cap hanging platform is suitable for abutting against an inner end face of one end of the outer sleeve based on the potential energy of the spring, and the lancet outlet is provided with an opening passage that allows the lancet cap hanging platform to pass through only in a specific direction; and the abutment between the supporting surface and the abutting member and the abutment between the lancet cap hanging platform and the inner end face of the outer sleeve jointly prevent the inner sleeve from moving in the axial direction toward the interior of the outer sleeve.

Optionally, a positioning surface adjacent to the lancet core guide portion is arranged in the outer sleeve, and the lancet core has an abutting end face that abuts against the positioning surface; and in the case that the lancet core abuts against the abutting member in the inner sleeve and the lancet core abuts against the outer sleeve, there is a predetermined distance between the abutting end face and the positioning surface in the axial direction.

### BRIEF DESCRIPTION OF THE DRAWINGS

Fig. 1 is a three-dimensional schematic diagram of a lancet core of a blood lancet according to an exemplary embodiment of the present disclosure;
Fig. 2A-Fig. 2E are a schematic front view, a top view, a section view, a section view and a space diagram of an inner sleeve of a blood lancet according to an exemplary embodiment of the present disclosure;
Fig. 3A-Fig. 3E are a schematic space diagram, a space diagram, a top view, a section view and a local amplified schematic diagram of an outer sleeve of a blood lancet according to an exemplary embodiment of the present disclosure;
Fig. 4 is a three-dimensional schematic diagram of an end cover of a blood lancet according to an exemplary embodiment of the present disclosure;
Fig. 5A is a schematic section view of a blood lancet according to an exemplary embodiment of the present disclosure, wherein a lancet cap is not removed from a lancet core;
Fig. 5B is a schematic section view of a blood lancet according to an exemplary embodiment of the present disclosure, wherein the lancet cap is not removed from the lancet core, a supporting surface abuts against an abutting member, and a lancet cap hanging platform abuts against an inner end face of the outer sleeve;
Fig. 6 is a schematic section view of a blood lancet according to an exemplary embodiment of the present disclosure, wherein the lancet cap has been removed from the lancet core, and the lancet core abuts against the outer sleeve;
Fig. 7 is a schematic section view of a blood lancet according to an exemplary embodiment of the present disclosure, wherein the lancet core is in a lunching process, and a lancet tip is not punctured from a lancet outlet;
Fig. 8 is a schematic section view of a blood lancet according to an exemplary embodiment of the present disclosure, wherein the lancet core is in the lunching process, and the lancet tip is punctured from the lancet outlet;
Fig. 9A is a schematic sectional view of a blood lancet according to an exemplary embodiment of the present disclosure, wherein the blood collection operation of the blood lancet has been completed;
Fig. 9B is a schematic sectional view of a blood lancet according to an exemplary embodiment of the present disclosure, wherein the blood lancet is in a state shown in Fig. 5A;
Fig. 10A and Fig. 10B are schematic diagrams exemplarily showing a process in which a first protrusion passes cover a first blocking portion.

### DETAILED DESCRIPTION OF THE EMBODIMENTS

The technical solutions of the present disclosure will be further described below in detail through embodiments and in conjunction with the drawings. In the specification, the same or similar reference signs indicate the same or similar components. The following descriptions of the embodiments of the present disclosure with reference to the drawings are intended to explain the general inventive concept of the present disclosure, and should not be understood as a limitation to the present disclosure.

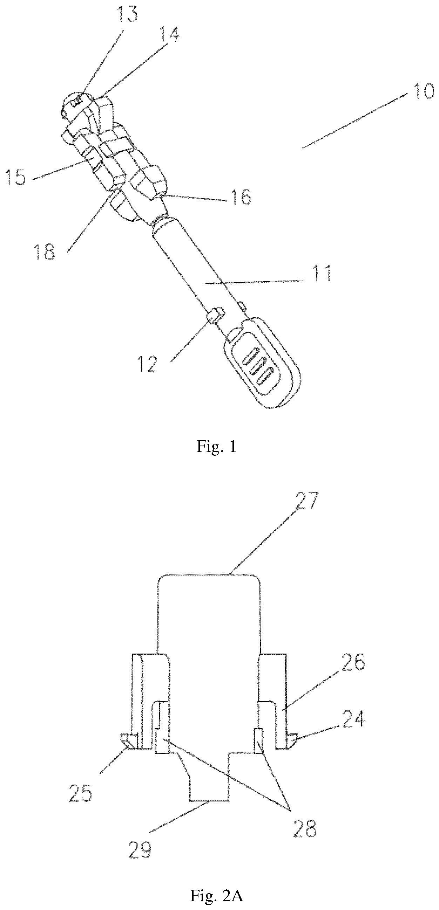

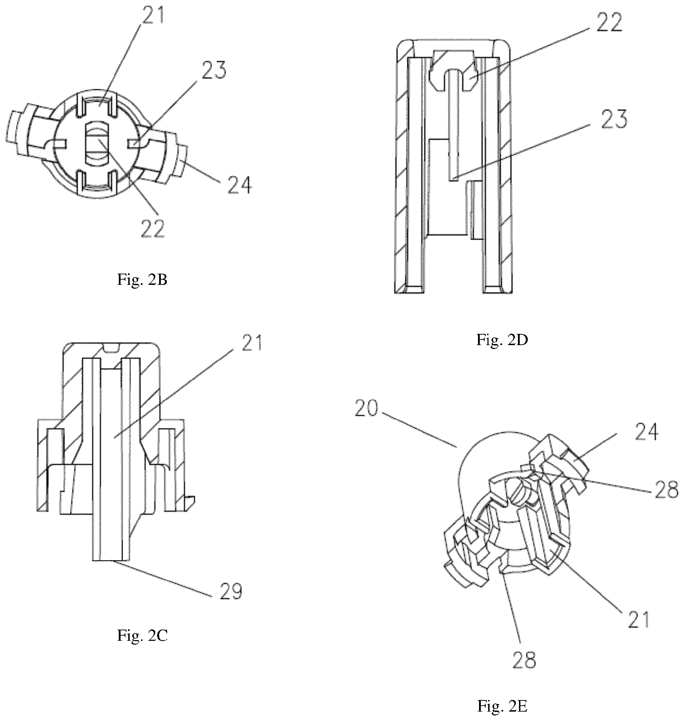

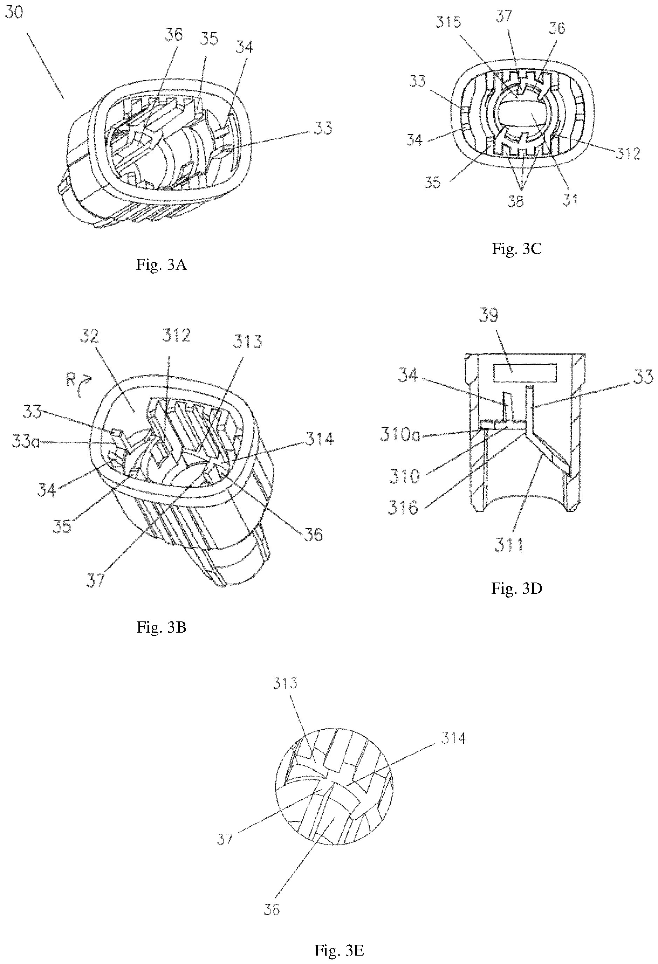

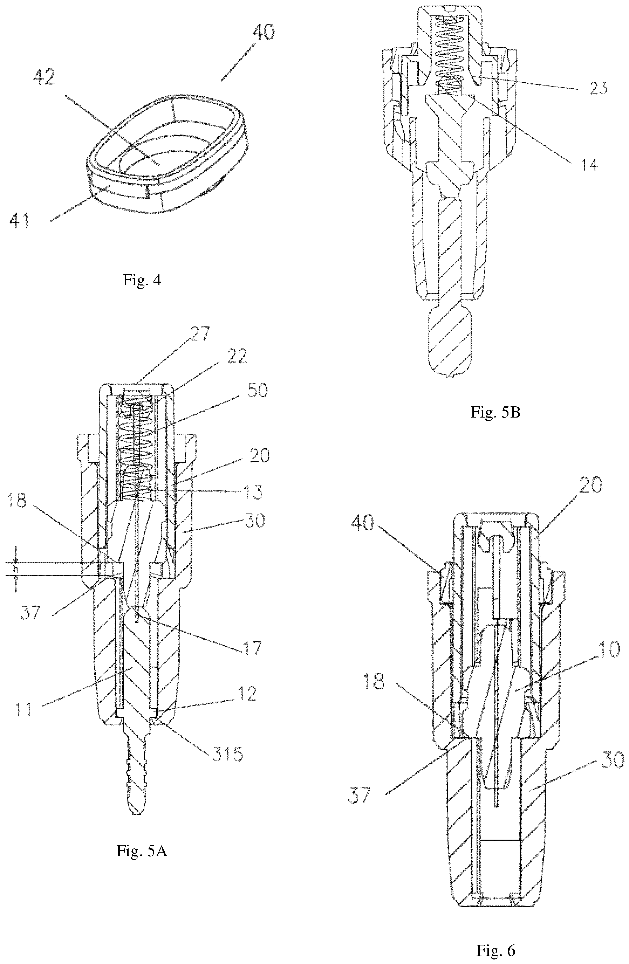

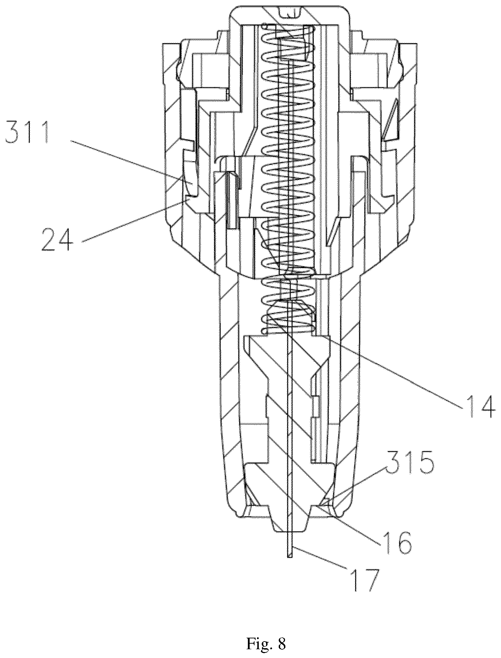

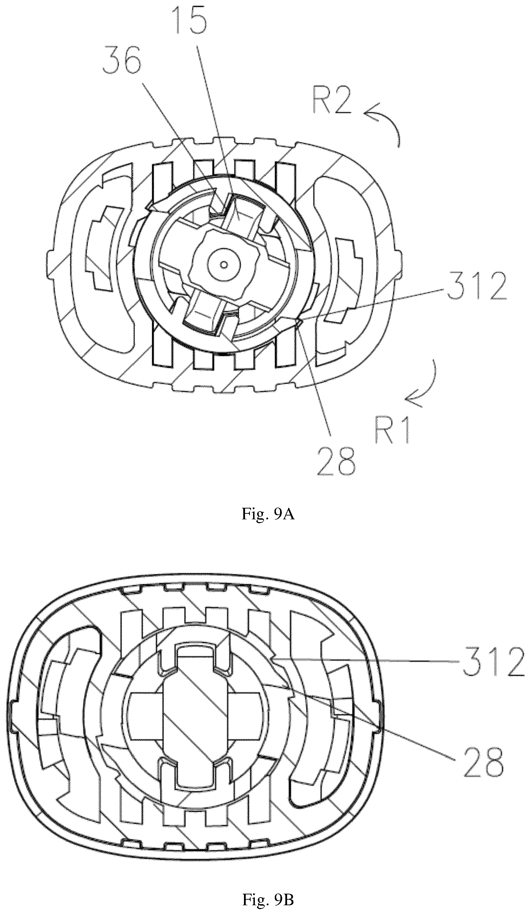

At first, the blood lancet according to the exemplary embodiments of the present disclosure will be described in detail with reference to the drawings. It should be noted that, for the clarity of display, some features or components in the drawings are not specifically shown. As shown in the figures, in a specific embodiment, the blood lancet according to the present disclosure includes:
an outer sleeve 30, provided with a lancet outlet 31 (as shown in Fig. 3C) at one end (in the present disclosure, one end is the front end, that is, the end part close to the blood collection skin), and an accommodating port 32 at the other end (in the present disclosure, the other end is the rear end, that is, the end part away from the blood collection skin), wherein a lancet core guide portion 36 (for example, referring to Fig. 3A to 3C and Fig. 3E) is arranged in the outer sleeve;
an inner sleeve 20, wherein one end of the inner sleeve enters the outer sleeve 30 from the accommodating port, and the other end of the inner sleeve is located at the outside of the accommodating port, for example, referring to Fig. 5A;
a lancet core 10, provided with a lancet body, wherein the lancet core is arranged at one end of the inner sleeve so as to rotate along the circumferential direction with the inner sleeve; and
a spring 50, arranged between the lancet core 10 and the inner sleeve 20, as shown in Fig. 5A, and provides power for the lancet core to move axially along the lancet core guide portion toward the lancet outlet,
wherein:
   based on the axial movement of the inner sleeve 20 in the direction toward the lancet outlet 31, the inner sleeve rotates from a first circumferential position relative to the outer sleeve 30 to a second circumferential position;
   at the first circumferential position (for example, corresponding to Fig. 5A), the outer sleeve 30 abuts against the lancet core 10 to prevent the lancet core from launching, and at the second circumferential position (for example, corresponding to Fig. 8), the lancet core 10 is suitable for entering the lancet core guide portion 36 for launching (including entering the lancet core guide portion); and
   as shown in Fig. 2A and Fig. 2E, a first protrusion 28 is arranged on the radial outer side of a cylinder body of the inner sleeve 20, as shown in Fig. 3B and Fig. 3C, a first blocking portion 312 is arranged in the outer sleeve 30, when the inner sleeve 20 is at the first circumferential position, the first protrusion 28 is located on one side of the first blocking portion 312 in the circumferential direction, for example, referring to Fig. 9B, when the inner sleeve 20 is at the second circumferential position, the first protrusion 28 is located on the other side (for example, referring to Fig. 9A) of the first blocking portion 312 in the circumferential direction, and the first blocking portion 312 prevents the first protrusion 28 located on the other side of the first blocking portion 312 from moving circumferentially to the one side of the first blocking portion 312 (in Fig. 9A, preventing the first protrusion 28 from rotating along a direction R2).

In an exemplary embodiment of the present disclosure, the lancet core 10 is provided with a supporting surface 14 (for example, referring to Fig. 8) at the end where the spring is provided, and an abutting member 23 is arranged in the inner sleeve 20 (for example, referring to Fig. 2B and Fig. 2D, the abutting member 23 and the supporting surface 14 are suitable for abutting against each other, for example, referring to Fig. 5B); the lancet core 10 further includes a lancet cap 11 arranged on the lancet body, as shown in Fig. 5A, the lancet cap 11 passes through the outer sleeve 30 through the lancet outlet 31, the lancet cap 11 is provided with a lancet cap hanging platform 12, the lancet cap hanging platform is suitable for abutting against an inner end face 315 of one end of the outer sleeve based on the potential energy of the spring, as shown in Fig. 3C, and the lancet outlet 31 is provided with an opening passage that allows the lancet cap hanging platform to pass through only in a specific direction; and the abutment between the supporting surface 14 and the abutting member 23 and the abutment between the lancet cap hanging platform 12 and the inner end face 315 of the outer sleeve jointly prevent the inner sleeve from moving in the axial direction toward the interior of the outer sleeve or limiting the inner sleeve 20 at a position relative to the outer sleeve 30 in the axial direction of the outer sleeve.

In the embodiment of the present disclosure, because of the abutment between the supporting surface 14 and the abutting member 23 and the abutment between the lancet cap hanging platform 12 and the inner end face 315, the inner sleeve 20 cannot move relative to the outer sleeve 30 in the axial direction. This can play a role of preventing false triggering.

In the present disclosure, in the axial direction, the abutting member in the inner sleeve abuts against the lancet core to apply an abutting force toward the direction of the lancet outlet, a component in the outer sleeve abuts against the lancet core to apply an abutting force toward the accommodating port, and the lancet core abuts against the abutting member in the inner sleeve and abuts against the outer sleeve at the same time to prevent the inner sleeve from moving toward the lancet outlet in the axial direction.

In the present disclosure, limiting the position of the inner sleeve 20 relative to the outer sleeve 30 is not limited to the embodiment shown in the drawings of the present disclosure. For example, in Fig. 2D, the abutting member 23 takes an elongated shape extending in the axial direction and is connected to the inner end face of the inner sleeve. However, the abutting member 23 can also be arranged on the cylinder wall of the inner sleeve, and the abutting member 23 is not limited to be strip-shaped. As another example, in Fig. 5A, the abutment between the lancet core and the outer sleeve is realized by using the abutment between the lancet cap hanging platform 12 and the inner end face 315, but the present disclosure is not limited thereto, it can also be other structures that can cooperate with the structure in the outer sleeve on the lancet core. All structures that can abut the lancet core against the lancet outlet by using the inner sleeve 20 and abut the lancet core against the accommodating port by using the outer sleeve to prevent the inner sleeve from moving further into the outer sleeve shall fall within the protection scope of the present disclosure.

Although not shown, the abutment between the lancet core and the outer sleeve can also be realized by using a positioning surface 37 and an abutting end face 18 in Fig. 5A.

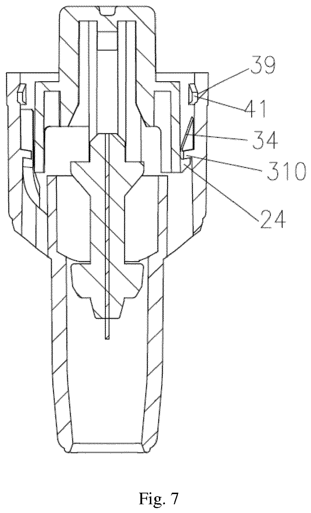

As shown in Fig. 3D, the outer sleeve 30 is provided with a second blocking portion 310, as shown in Fig. 2A, the other end (that is, the lower end in the figure) of the inner sleeve 20 is provided with a second protrusion 24 protruding radially outward, and as shown in Fig. 7, the second blocking portion 310 is suitable for cooperating with the second protrusion 24 to prevent the second protrusion 24 from moving in the direction from the lancet outlet to the accommodating port. In this way, the inner sleeve 20 can be prevented from falling off from the outer sleeve 30.

The cooperation between the second protrusion 24 and the second blocking portion 310 can correspond to the snap fit between the inner sleeve 20 and the outer sleeve 30, therefore, after the inner sleeve 20 enters the outer sleeve 30 from the accommodating port axially, the inner sleeve 20 is impossible or difficult to be pulled out or withdrawn from the accommodating port of the outer sleeve 30.

In a specific embodiment, the second blocking portion 310 includes a circumferential protrusion arranged on the inner wall of the outer sleeve 30, the circumferential protrusion protrudes radially inward, and the circumferential protrusion has a blocking surface 310a that is suitable for cooperating with the second protrusion 24 to prevent the second protrusion 24 from moving in the direction from the lancet outlet to the accommodating port, as shown in Fig. 3D.

It should be noted that the structure of the second blocking portion 310 is not limited to the circumferential protrusion shown in the figure. Although not shown, the second blocking portion can also be a recess or a slot formed in the inner wall of the outer sleeve 30, and the recess or the slot can cooperate with the second protrusion 24 to prevent the second protrusion 24 from moving in the direction from the lancet outlet to the accommodating port.

In a more specific embodiment, as shown in Fig. 2A to Fig. 2C, the other end of the inner sleeve 20 is provided with an elastic arm 26, and the end part of the elastic arm 26 is provided with the second protrusion 24 protruding radially outward from the elastic arm. In the case that the second blocking portion is a circumferential protrusion, the second protrusion 24 is suitable for passing cover the second blocking portion 310 in the direction from the accommodating port to the lancet outlet.

In another embodiment of the present disclosure, the radial elastic deformation can be provided by the second blocking portion in the outer sleeve. In other words, the second blocking part can be extruded radially outward by the second protrusion 24, and after the second protrusion 24 passes cover the second blocking portion in the axial direction, the second blocking portion resets, thereby preventing the second protrusion from departing from the outer sleeve in the direction from the lancet outlet to the accommodating port. More specifically, the second blocking portion includes an inclined cantilever, which is elastically deformable radially outward, one end of which is connected to the inner wall of the outer sleeve, and the other end of which extends obliquely inward toward the lancet outlet, the second protrusion extrudes the inclined cantilever radially outward during passing through the inclined cantilever, and after the second protrusion passes through the inclined cantilever, the other end of the inclined cantilever moves radially inward to prevent the second protrusion from moving in the direction from the accommodating port to the lancet outlet. The above solution is also within the protection scope of the present disclosure.

In the present disclosure, the cooperation between the second protrusion and the second blocking portion can be snap fit. As those skilled in the art can understand, this snap fit is used for preventing the second protrusion from departing from the outer sleeve in the direction from the lancet outlet to the accommodating port on one hand, and on the other hand, it can also provide a rotation movement space for the circumferential rotation of the second protrusion.

In the illustrated embodiment, the second blocking portion is arranged in the outer sleeve 30 and protrudes radially inward, but the present disclosure is not limited thereto. The second blocking portion includes a groove or a slot formed in the inner wall of the outer sleeve or a through hole passing through the cylinder wall of the outer sleeve, and the groove or the slot or the through hole is suitable for cooperating with the second protrusion to prevent the second protrusion from moving in the direction from the accommodating port to the lancet outlet. In the case that the second blocking portion is a groove, a recess or a through hole, the second protrusion 24 is suitable for moving radially outward to enter the groove, the recess or the through hole, and these are all within the protection scope of the present disclosure.

In the present disclosure, the second protrusion only needs to be able to cooperate with the second blocking portion to prevent the second protrusion, so that the inner sleeve departs from the outer sleeve in the direction from the lancet outlet to the accommodating port. The second protrusion can be in the form of a hook or other forms, which are all within the protection scope of the present disclosure.

In the case that the inner sleeve needs to rotate relative to the outer sleeve, snap fit structures formed by the second protrusion and the second blocking portion, as long as being able to prevent the inner sleeve from being pulled out from the outer sleeve and allow the inner sleeve to rotate at an angle relative to the outer sleeve, are all within the protection scope of the present disclosure.

When the inner sleeve 20 is assembled to the outer sleeve 30, in order to guide the axial movement of the second protrusion 24 and to conveniently insert the inner sleeve 20 into the outer sleeve 30, as shown in Fig. 3A to Fig. 3D, a cooperation guide inclined surface 34 is arranged in the outer sleeve 30, and the second protrusion 24 is suitable for abutting against the cooperation guide inclined surface 34 to drive the elastic arm 26 to deform radially inward.

In a further embodiment, as shown in Fig. 3B, the second protrusion 24 is provided with a second convex inclined surface 25 that cooperates with the cooperation guide inclined surface 34.

In an exemplary embodiment, the position of the second protrusion 24 can also be limited in the circumferential direction when the inner sleeve 20 is assembled to the outer sleeve 30. As shown in Fig. 3B, a first limiting portion 33 protruding radially inward is arranged in the outer sleeve 30, and the first limiting portion 33 is suitable for cooperating with one side face of the second protrusion 24 during the engagement process of the second protrusion 24 and the cooperation guide inclined surface 34. It should be pointed out that the cooperation here includes contact and non-contact situations, which means that the position of the second protrusion 24 can be limited in the circumferential direction. More specifically, as shown in Fig. 3B, a side wall 33a of the first limiting portion 33 can block the side face of the second protrusion 24.

The first limiting portion 33 can limit the position of the second protrusion 24 in the circumferential direction on one side in the circumferential direction. In a further embodiment, it can also limit the position of the second protrusion 24 on the other side in the circumferential direction. As shown in Fig. 3A to Fig. 3C, a second limiting portion 35 protruding radially inward is arranged in the outer sleeve 30, and the second limiting portion 35 is suitable for cooperating with one side face of the elastic arm 26, so that the elastic arm 26 and the second protrusion 24 are located between the first limiting portion 33 and the second limiting portion 35 in the circumferential direction during the engagement process of the second protrusion 24 and the cooperation guide inclined surface 34. By using the first limiting portion and the second limiting portion, it can be ensured that the inner sleeve 20 is installed at the correct position. Both the first limiting portion and the second limiting portion can be rib-shaped or convex rib-shaped.

In an embodiment of the present disclosure, the blood lancet further includes a circumferential inclined extension portion (corresponding to the third blocking portion), the second blocking portion 310 and a circumferential extension blocking portion are arranged in sequence along the circumferential direction, the second protrusion 24 is suitable for moving circumferentially from the second blocking portion to a circumferential inclined extension blocking portion, and the circumferential inclined extension blocking portion is suitable for preventing the second protrusion 24 from moving in the direction from the lancet outlet to the accommodating port. As shown in Fig. 3B and Fig. 3D, in an exemplary embodiment of the present disclosure, the outer sleeve 30 is provided with a circumferential inclined extension blocking surface 311 (the circumferential inclined extension blocking portion includes the circumferential inclined extension blocking surface 311) from the first limiting portion 33 in the circumferential direction from the cooperation guide inclined surface 34 to the first limiting portion 33, and the circumferential inclined extension blocking surface is suitable for preventing the second protrusion 24 from moving in the direction from the lancet outlet to the accommodating port (that is, the upward direction in Fig. 3B), after the second protrusion 24 moves in the circumferential direction (schematically as the R direction in Fig.3B) from the cooperation guide inclined surface 34 to the first limiting portion 33 to cross the first limiting portion 33. In this way, even if the inner sleeve 20 rotates relative to the outer sleeve 30, since the second protrusion 24 is blocked by the extension blocking surface 311, the inner sleeve 20 can be prevented from withdrawing from the outer sleeve 30.

In one embodiment, as shown in Fig. 3D, an axial extension blocking surface 316 is arranged between the circumferential inclined extension blocking surface 311 and the blocking surface 310a, one end of the axial extension blocking surface 316 is connected to the circumferential inclined extension blocking surface 311, and the other end thereof is connected to the blocking surface 310a.

Because of the presence of the axial extension blocking surface 316, the second protrusion 24 cannot move circumferentially toward the circumferential inclined extension blocking surface 311 in the case that the inner sleeve is not further pressed. In other words, in the state shown in Fig. 5A, the inner sleeve 20 cannot be rotated toward the circumferential inclined extension blocking surface 311.

As shown in Fig. 5A and Fig. 5B, in the case that the supporting surface 14 abuts against the abutting member 23 and the lancet cap hanging platform 12 abuts against the inner end face 315 of the outer sleeve, there is a predetermined distance h between an abutting end face 18 and a positioning surface 37 of the lancet core 10 in the axial direction. In the case that a lancet cap removed, as shown in Fig. 6, the abutting end face 18 abuts against the positioning surface 37. The predetermined distance can play a role of preserving a safe distance. More specifically, under the support of the lancet core 10, the inner sleeve 20 cannot move further into the outer sleeve 30, thereby prevents the situation that before the lancet cap of the blood lancet is removed, the inner sleeve 20 is pressed to drive a guide member 15 mentioned below to enter the lancet core guide portion 36 in advance, such that the blood lancet is launched in advance after the lancet cap is removed.

As can be understood, the predetermined distance h can provide a space for the further pressing movement of the inner sleeve 20, for example, there is a predetermined distance h in the axial direction between the supporting surface 14 and the abutting member 23, therefore when the inner sleeve 20 is pressed down, the supporting surface 14 will not prevent the abutting member 23 from moving downward.

As shown in Fig. 3E, the positioning surface 37 is an end face that forms a rib of the lancet core guide portion 36. Although not shown, the positioning surface 37 can also be a surface that is coplanar and in contact with the end face of the rib, and these are all within the protection scope of the present disclosure.

When the lancet core 10 rotates with the inner sleeve, the abutting end face 18 can enter the lancet core guide portion 36 from the positioning surface 37.

In an exemplary embodiment of the present disclosure, the spring 50 can be in a compressed state when the blood lancet is not used, for example, referring to Fig. 5A. In the case that the blood lancet is provided with only one spring and the spring is a compression spring, the spring 50 is arranged between the lancet core 10 (a mounting portion 13 for fixing one end of the spring 50 is arranged on the lancet core, referring to Fig. 1) and one end of the inner sleeve 20 in the inner sleeve 20, and the other end of the compression spring is fixed to the inner end face of one end of the inner sleeve 20 (a spring mounting portion 22 is arranged in the inner sleeve, referring to Fig. 2B). As those skilled in the art can understand, in the case that the compression spring only provides an elastic force to eject the lancet core, and there is another extension spring to pull the lancet core back, the compression spring can also only abut against the lancet core and the inner sleeve instead of fixing both ends thereof to the lancet core and inner sleeve; and in addition, a tension spring can also be arranged on the side close to the lancet outlet, and the tension spring provides an elastic force for ejecting the lancet body and an elastic force for pulling back the ejected lancet body. These are all within the protection scope of the present disclosure.

The structure of pressing the inner sleeve 20 downward to rotate the lancet core will be exemplarily described below with reference to the drawings.

As shown in Fig. 3B and Fig. 3E, the positioning surface 37 adjacent to the lancet core guide portion 36 is arranged in the outer sleeve 30, and as shown in Fig. 1 and Fig. 5A, the lancet core 10 has the abutting end face 18 that abuts against the positioning surface 37. As shown in Fig. 3B and Fig. 3E, a rotation guide portion 313 is arranged in the outer sleeve 30, and one end of the inner sleeve 20 is provided with a rotation cooperation portion 29 that cooperates with the rotation guide portion; and based on the axial movement of the inner sleeve 20 in the direction toward the lancet outlet, the rotation cooperation portion 29 cooperates with the rotation guide portion 313 to cause the inner sleeve 20 to rotate in the circumferential direction, and based on the rotation, the abutting end face 18 is suitable for leaving the positioning surface 37 so as to enter the lancet core guide portion 36.

In the present disclosure, top triggering is used, that is, the lancet body is ejected by pressing the inner sleeve 20 at the top. Correspondingly, the inner sleeve 20 can be considered as a button for triggering.

As those skilled in the art can understand, the inner sleeve 20 can move axially along the outer sleeve, and after the axial movement is in place, the inner sleeve 20 can rotate at least one angle relative to the outer sleeve 30 (this angle can allow the abutting end face 18 to leave positioning surface 37 so as to enter the lancet core guide portion 36).

As shown in Fig. 2A to Fig. 2C, the inner sleeve 20 is provided with a guide groove 21; and as shown in Fig. 1, the lancet core 10 is provided with a guide member 15 that cooperates with the guide groove 21, the guide member 15 is arranged in the guide groove 21, and the guide groove 21 is suitable for driving the guide member 15 so as to drive the lancet core 10 to rotate along the circumferential direction.

As shown in Fig. 3E, the rotation guide portion 313 includes a circumferential inclined surface. As shown in Fig. 2C, the end part of the guide groove 21 constitutes the rotation cooperation portion 29 to be suitable for being engaged with the circumferential inclined surface.

As shown in Fig. 3B, the outer sleeve 30 is further provided with a circumferential plane 314 that is in contact with the circumferential inclined surface. In the case that the rotation cooperation portion moves from the circumferential inclined surface to the circumferential plane 314 based on the rotation of the inner sleeve, the guide groove 21 and the lancet core guide portion 36 are aligned in the axial direction to allow the guide member 15 to move from the guide groove 21 to the lancet core guide portion 36.

As shown in Fig. 3B and Fig. 3E, the rotation guide portion 313 includes an inclined rib arranged in the outer sleeve 30, and the inclined rib protrudes radially inward and has the circumferential inclined surface. In a further embodiment, as shown in Fig. 3E, the circumferential inclined surface is located on the outer side of the lancet core guide portion 36 in the radial direction.

It should be pointed out that the rotation guide portion is not limited to the embodiment shown in the figure. For example, the rotation cooperation portion cannot be arranged at the end part of the guide groove, it can also include a cooperation flange projecting radially outward from the inner sleeve (although not shown, for example, the cooperation flange can be arranged on the outer wall of the inner sleeve, for example, a position opposite to the guide groove), and the cooperation flange constitutes the rotation cooperation portion and is adapted to be engaged with the circumferential inclined surface. This is also within the protection scope of the present disclosure.

Although not shown, the rotation guide portion can also be an inclined guide groove; and the rotation cooperation portion is a cooperation protrusion protruding radially outward from the inner sleeve, and the cooperation protrusion is suitable for moving in the inclined guide groove.

As shown in Fig. 4 and Fig. 6, the blood lancet further includes an end cover 40, the end cover 40 is provided with a through hole 42, and the other end of the inner sleeve 20 is exposed from the through hole to the outside of the outer sleeve 30; and the end cover 40 is detachably connected to the accommodating port of the outer sleeve so as to cover the accommodating port.

In a specific exemplary embodiment, as shown in Fig. 4, the end cover 40 is further provided with a fastener 41, such as a rib; as shown in Fig. 3D, a slot 39 is formed at the accommodating port of the outer sleeve, and the slot 39 cooperates with the fastener 41 to detachably dispose the end cover 40 on the outer sleeve, referring to Fig. 7. After the end cover 40 is assembled, it can cover the gap between the inner sleeve 20 and the outer sleeve 30, thereby playing the role of beautification and sanitation.

As shown in the drawings, the inner sleeve 20 has a cylindrical main body. As shown in Fig. 3B and Fig. 3C, a circumferential limiting portion 38 is arranged in the outer sleeve 30, and the inner diameter defined by the circumferential limiting portion 38 is slightly larger than the outer diameter of the cylindrical main body of the inner sleeve. The circumferential limiting portion 38 is conducive to maintaining the stability of the inner sleeve during axial movement.

In the present disclosure, in order to prevent the secondary use of the blood lancet, it is necessary to ensure that, after the blood lancet is used, even if the inner sleeve 20 is pressed, it cannot be triggered to eject the lancet tip.

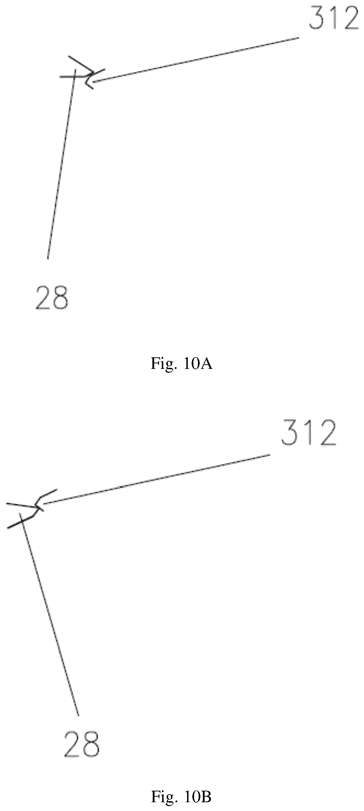

As shown in Fig. 2A and Fig. 2E, the first protrusion 28 is arranged on the radial outer side of the cylinder body of the inner sleeve 20; and as shown in Fig. 3B and Fig. 3C, the first blocking portion 312is arranged on the inner wall of the outer sleeve 30. Fig. 10A and Fig. 10B are schematic diagrams exemplarily showing a process in which the first protrusion 28 passes cover the first blocking portion 312. Fig. 9A shows a state in which the first blocking portion 312 abuts against and cooperates with the first protrusion 28 in the state as shown in Fig. 8. Fig. 9B is a schematic sectional view of a blood lancet according to an exemplary embodiment of the present disclosure, wherein the blood lancet is in a state shown in Fig. 5A. In Fig. 9B, the positional relationship between the blocking portion 312 and the protrusion 28 corresponds to that the inner sleeve 20 is at the first circumferential position relative to the outer sleeve 30, while in Fig. 9A, the positional relationship between the blocking portion 312 and the protrusion 28 corresponds to the that the inner sleeve 20 is at the first circumferential position relative to the outer sleeve 30.

The first protrusion 28 and the first blocking portion 312 cooperate in the circumferential direction (referring to Fig. 9A and Fig. 9B), so that after the inner sleeve 20 rotates relative to the outer sleeve 30 to allow the lancet tip 17 to eject, the first protrusion 28 passes cover the first blocking portion 312 (from Fig. 10A to Fig. 10B) during the rotation process of the inner sleeve, thereby transforming the relative position relationship from Fig. 9B to the relative position relationship shown in Fig. 9A. In Fig. 9A, the abutment cooperation between the first blocking portion 312 and the first protrusion 28 prevents the inner sleeve 20 from rotating in a reverse direction (the direction R2 in Fig. 9A) to the circumferential position before the lancet tip is ejected, thereby preventing the secondary use of the blood lancet.

In the present disclosure, as long as the cooperation between the first protrusion 28 and the first blocking portion 312 can allow the first blocking portion 312 to prevent the first protrusion 28 from passing cover in a second circumferential direction R2 opposite to a first circumferential direction R1 after passing cover the first blocking portion 312 in the first circumferential direction R1, it is not limited to the structure shown in the drawings of the present disclosure, and other structural changes are all within the protection scope of the present disclosure.

In the present disclosure, based on the cooperation between the third blocking portion (including the circumferential inclined extension blocking surface 311) and the second protrusion 24, after the blood collection operation of the blood lancet, the inner sleeve 20 can be prevented from departing from the outer sleeve 30.

In this way, after the blood collection operation of the blood lancet, in the circumferential direction, the inner sleeve 20 is blocked by the first blocking portion 312, and in the axial direction, the inner sleeve 20 is blocked by the blocking surface 311, thereby further preventing the secondary use of the blood lancet.

As shown in Fig. 1 and Fig. 8, the lancet core 10 has a stop surface 16, and after the lancet core 10 is ejected, the stop surface 16 abuts against the inner end face 315 of the outer sleeve 30 to limit the penetration depth of the lancet tip. However, the stop surface 16 can be not provided, and the end face 18 is used for abutting against the inner end face 315.

The assembly of the blood lancet of the present disclosure is exemplarily described below.

Firstly, the other end of the spring 50 is installed on the protrusion 22 at the inner end face of the inner sleeve 20 (referring to Fig. 2B).

Secondly, the lancet core 10 is inserted into the inner sleeve 20, one end of the spring 50 is connected to the mounting protrusion 13 (referring to Fig. 1) of the lancet core 10, and the guide member 15 is driven to enter the guide groove 21 of the inner sleeve 20.

Thirdly, a combination of the lancet core 10 and the inner sleeve 20 is inserted into the outer sleeve 30 from the accommodating port of the outer sleeve 30, during the insertion, the second protrusion 24 on the inner sleeve 20 cooperates with the guide inclined surface 34 so as to move radially inward on the basis of the elasticity of the elastic arm 26, after the second protrusion 24 passes cover the second blocking portion 310, the radial position of the second protrusion 24 rebounds based on the elasticity of the elastic arm 26, and the second protrusion 24 is prevented by the blocking surface 310a from moving upward, therefore the inner sleeve 20 is snap-fitted to the outer 30. Due to the elastic pressure of the spring 50, the lancet cap hanging platform 12 of the lancet cap 11 abuts against the inner end face 315 at the lancet outlet of the outer sleeve, as shown in Fig. 5A. In addition, during the further movement of the inner sleeve 20 into the outer sleeve 30, the abutting member 23 in the outer sleeve abuts against the supporting surface 14 on the lancet core 10. When the abutting member 23 abuts against the supporting surface 14 and the lancet cap hanging platform 12 abuts against the inner end face 315, the inner sleeve 20 cannot move further into the outer sleeve, and as shown in Fig. 5A, there is also a predetermined distance between the abutting end face 18 and the positioning faces 37. In addition, when the second protrusion 24 is engaged with the blocking surface 310a, the second protrusion 24 is blocked by the axial extension blocking surface 316 in the circumferential direction.

Finally, the end cover 40 is installed on the outer sleeve 30.

In the assembled blood lancet, the relative positions of the first blocking portion 312 and the first protrusion 28 in the circumferential direction are shown in Fig. 9B.

The blood collection operation of the blood lancet according to the present disclosure is illustrated below.

Firstly, the lancet cap 11 is removed from the blood lancet (for example, by screwing) to expose the lancet head 17 or the lancet tip. After the lancet cap 11 is removed, the inner sleeve 20 loses the support of the lancet cap hanging platform 12 on both sides of the lancet cap. At this time, because there is no lancet cap hanging platform 12 abutting against the inner end face 315 at the lancet outlet of the outer sleeve, under the elastic force of the spring 50, the abutting end face 18 moves axially based on the elastic force of the spring to abut against the positioning surface 37, as shown in Fig. 6. At this time, the blood lancet is in a state to be launched, there is a distance between the abutting member 23 in the inner sleeve and the supporting surface 14 on the lancet core 10 to allow the inner sleeve 20 to move further axially into the outer sleeve, and the second protrusion 24 is blocked by the axial extension blocking surface 316 in the circumferential direction.

Secondly, the top end 27 of the inner sleeve 20 is pressed to cause the inner sleeve 20 to move axially inward, so that before the end part of the guide groove 21 of the inner sleeve 20 serving as the guide cooperation portion 29 is in contact with the inclined surface 313, the second protrusion 24 is free from the blocking of the axial extension blocking surface 316 in the circumferential direction.

Thirdly, the inner sleeve 20 is further pressed, the guide cooperation portion 29 moves axially to the inclined surface on the inclined rib serving as the rotation guide portion 313, so that the guide cooperation portion 29 slides on the inclined surface, in this way, the inner sleeve 20 rotates while moving axially, the inner sleeve 20 rotates to drive the lancet core 10 to rotate, so that the abutting end face 18 of the lancet core 10 leaves the positioning surface 37 and enters the starting end of the lancet core guide portion 36, and then the lancet core 10 is ejected under the action of the elastic force of the spring 50, as shown in Fig. 7. In the case that the guide cooperation portion 29 moves from the circumferential inclined surface (corresponding to 313) to the circumferential plane 314 based on the rotation of the inner sleeve 20, the guide groove 21 is aligned with the lancet core guide portion 36 in the axial direction to allow the guide member 15 to move from the guide groove 21 to the lancet core guide portion 36.

In the process of further pressing the inner sleeve 20, based on the rotation of the inner sleeve 20, the first protrusion 28 of the inner sleeve 20 gradually passes cover the first blocking portion 312, as shown in Fig. 10A and Fig. 10B, until the state shown in Fig. 9A.

In addition, as shown in Fig. 8, the lancet head 17 of the blood lancet is exposed at the outside of the lancet outlet and is used for puncturing the skin of the patient. As shown in Fig. 8, when the lancet tip 17 is pierced, the second protrusion 24 has already turned below the circumferential inclined blocking surface 311 to abut against it, thereby preventing the inner sleeve from moving toward the outside of the outer sleeve or moving upward.

Subsequently, due to the tension of the spring 50, the lancet head 17 retracts back into the shell to complete blood collection.

After the blood collection operation, the relative positions of the first blocking portion 312 and the first protrusion 28 in the circumferential direction are shown in Fig. 9A.

According to the blood lancet of the present disclosure, the launching of the lancet body is triggered by pressing the inner sleeve at the top of the blood lancet, which not only avoids the problem that the blood lancet penetrates too deeply into the skin of the patient due to the overall pressing, but also simplifies the blood collection operation.

Although the embodiments of the present disclosure have been shown and described, those of ordinary skill in the art can understand that changes can be made to these embodiments without departing from the principle of the present disclosure, and the scope of the present disclosure is determined by the appended claims.

## Claims

1. A blood lancet, comprising:
an outer sleeve (30), provided with a lancet outlet (31) at one end and an accommodating port at the other end, wherein a lancet core guide portion (36) is arranged in the outer sleeve;
an inner sleeve (20), wherein one end of the inner sleeve enters the outer sleeve from the accommodating port, and the other end of the inner sleeve is located at the outside of the accommodating port;
a lancet core (10), provided with a lancet body, wherein the lancet core is arranged at one end of the inner sleeve so as to rotate along the circumferential direction with the inner sleeve; and
a spring (50), arranged between the lancet core and the inner sleeve, and provides power for the lancet core to move axially along the lancet core guide portion toward the lancet outlet,
wherein:
based on the axial movement of the inner sleeve in the direction toward the lancet outlet, the inner sleeve rotates relative to the outer sleeve from a first circumferential position to a second circumferential position;
at the first circumferential position, the outer sleeve abuts against the lancet core to prevent the lancet core from launching, and at the second circumferential position, the lancet core is suitable for entering the lancet core guide portion for launching; and
a first protrusion (28) is arranged on the radial outer side of a cylinder body of the inner sleeve, a first blocking portion (312) is arranged in the outer sleeve, when the inner sleeve is at the first circumferential position, the first protrusion is located on one side of the first blocking portion in the circumferential direction; and when the inner sleeve is at the second circumferential position, the first protrusion is located on the other side of the first blocking portion in the circumferential direction, and the first blocking portion prevents the first protrusion located on the other side of the first blocking portion from moving circumferentially to the one side of the first blocking portion.

2. The blood lancet according to claim 1, wherein:
the outer sleeve is provided with a second blocking portion (310) and a third blocking portion (312), the second blocking portion and the third blocking portion are arranged in sequence along the circumferential direction, and the third blocking portion is a circumferential inclined extension blocking portion;
the other end of the inner sleeve is provided with a second protrusion (24) protruding radially outward, and both the second blocking portion and the third blocking portion are suitable for cooperating with the second protrusion to prevent the second protrusion from moving in the direction from the lancet outlet to the accommodating port; and
when the inner sleeve is at the first circumferential position, the second protrusion abuts against the second blocking portion, and when the inner sleeve is at the second circumferential position, the second protrusion abuts against the third blocking portion.

3. The blood lancet according to claim 2, wherein:
the second blocking portion comprises a circumferential protrusion arranged on the inner wall of the outer sleeve, the circumferential protrusion protrudes radially inward, and the circumferential protrusion has a blocking surface (310a) that is suitable for cooperating with the second protrusion to prevent the second protrusion from moving in the direction from the lancet outlet to the accommodating port.

4. The blood lancet according to claim 3, wherein:
the third blocking portion comprises a circumferential inclined extension blocking surface (311), an axial extension blocking surface (316) is arranged between the circumferential inclined extension blocking surface and the blocking surface (310a), one end of the axial extension blocking surface is connected to the circumferential inclined extension blocking surface, and the other end thereof is connected to the blocking surface.

5. The blood lancet according to claim 2, wherein:
the other end of the inner sleeve is provided with an elastic arm (26), and the end part of the elastic arm is provided with the second protrusion protruding radially outward from the elastic arm.

6. The blood lancet according to claim 5, wherein:
a cooperation guide inclined surface (34) is arranged in the outer sleeve, and the second protrusion is suitable for abutting against the cooperation guide inclined surface to drive the elastic arm to deform radially inward, further alternatively, the second protrusion is provided with a second convex inclined surface that cooperates with the cooperation guide inclined surface.

7. The blood lancet according to claim 2, wherein:
the second blocking portion comprises a groove or a slot formed in the inner wall of the outer sleeve or a through hole passing through the cylinder wall of the outer sleeve, and the groove or the slot or the through hole is suitable for cooperating with the second protrusion to prevent the second protrusion from moving in the direction from the accommodating port to the lancet outlet; or
the second blocking portion comprises an inclined cantilever which is elastically deformable radially outward and one end of which is connected to the inner wall of the outer sleeve while the other end of which extends obliquely inward toward the lancet outlet, the second protrusion extrudes the inclined cantilever radially outward during passing through the inclined cantilever, and after the second protrusion passes through the inclined cantilever, the other end of the inclined cantilever moves radially inward to prevent the second protrusion from moving in the direction from the accommodating port to the lancet outlet.

8. The blood lancet according to any of claims 1-7, wherein:
a positioning surface (37) adjacent to the lancet core guide portion (36) is arranged in the outer sleeve, and the lancet core has an abutting end face (18) that abuts against the positioning surface;
a rotation guide portion (313) is arranged in the outer sleeve, and one end of the inner sleeve is provided with a rotation cooperation portion (29) that cooperates with the rotation guide portion; and
based on the axial movement of the inner sleeve in the direction toward the lancet outlet, the rotation cooperation portion (29) cooperates with the rotation guide portion (313) to cause the inner sleeve to rotate in the circumferential direction, and based on the rotation, the abutting end face (18) is suitable for leaving the positioning surface so as to enter the lancet core guide portion (36).

9. The blood lancet according to claim 8, wherein:
the inner sleeve is provided with a guide groove (21), the lancet core is provided with a guide member (15) that cooperates with the guide groove, the guide member is arranged in the guide groove, and the guide groove is suitable for driving the guide member so as to drive the lancet core to rotate along the circumferential direction, further alternatively, one end face of the guide member (15) constitutes the abutting end face (18).

10. The blood lancet according to claim 9, wherein:
the rotation guide portion (313) comprises a circumferential inclined surface;
the end part of the guide groove constitutes the rotation cooperation portion to be suitable for being engaged with the circumferential inclined surface; or the outer side of the inner sleeve is provided with a cooperation flange protruding radially outward, and the cooperation flange constitutes the rotation cooperation portion to be suitable for being engaged with the circumferential inclined surface.

11. The blood lancet according to claim 10, wherein:
the rotation guide portion (313) comprises an inclined rib arranged in the outer sleeve, and the inclined rib protrudes radially inward and has the circumferential inclined surface,
further alternatively,
the circumferential inclined surface is located on the outer side of the lancet core guide portion in the radial direction; and the outer sleeve is further provided with a circumferential plane (314) that is in contact with the circumferential inclined surface, and in the case that the rotation cooperation portion moves from the circumferential inclined surface to the circumferential plane based on the rotation of the inner sleeve, the guide groove and the lancet core guide portion are aligned in the axial direction to allow the guide member to move from the guide groove to the lancet core guide portion.

12. The blood lancet according to claim 8, wherein:
the rotation guide portion comprises an inclined guide groove;
the rotation cooperation portion comprises a cooperation protrusion that protrudes radially outward from the inner sleeve, and the cooperation protrusion is suitable for moving in the inclined guide groove.

13. The blood lancet according to any of claims 1-7, wherein:
in the axial direction, an abutting member in the inner sleeve abuts against the lancet core to apply an abutting force toward the direction of the lancet outlet, a component in the outer sleeve abuts against the lancet core to apply an abutting force toward the accommodating port, and the lancet core abuts against the abutting member in the inner sleeve and abuts against the outer sleeve at the same time to prevent the inner sleeve from moving toward the lancet outlet in the axial direction.

14. The blood lancet according to claim 13, wherein:
the lancet core is provided with a supporting surface (14) at the end where the spring is provided, and the abutting member and the supporting surface are suitable for abutting against each other;
the lancet core further comprises a lancet cap (11) arranged on the lancet body, the lancet cap passes through the outer sleeve through the lancet outlet, the lancet cap is provided with a lancet cap hanging platform (12) which is suitable for abutting against an inner end face (315) of one end of the outer sleeve based on the potential energy of the spring, and the lancet outlet is provided with an opening passage that allows the lancet cap hanging platform to pass through only in a specific direction; and
the abutment between the supporting surface (14) and the abutting member (23) and the abutment between the lancet cap hanging platform (12) and the inner end face (315) of the outer sleeve jointly prevent the inner sleeve from moving in the axial direction toward the interior of the outer sleeve.

15. The blood lancet according to claim 14, wherein:
a positioning surface (37) adjacent to the lancet core guide portion (36) is arranged in the outer sleeve, and the lancet core has an abutting end face (18) that abuts against the positioning surface; and
in the case that the lancet core abuts against the abutting member in the inner sleeve and the lancet core abuts against the outer sleeve, there is a predetermined distance between the abutting end face and the positioning surface in the axial direction.

## Patentansprüche

1. Blutlanzette, mit:
einer äußeren Hülse (30), die an einem Ende mit einem Lanzettenauslass (31) und am anderen Ende mit einer Aufnahmeöffnung versehen ist, wobei in der äußeren Hülse ein Lanzettenkernführungsabschnitt (36) angeordnet ist,
einer inneren Hülse (20), wobei ein Ende der inneren Hülse von der Aufnahmeöffnung in die äußere Hülse eintritt und das andere Ende der inneren Hülse an der Außenseite der Aufnahmeöffnung angeordnet ist,
einem Lanzettenkern (10), der mit einem Lanzettenkörper versehen ist, wobei der Lanzettenkern an einem Ende der inneren Hülse so angeordnet ist, dass er sich entlang der Umfangsrichtung mit der inneren Hülse dreht, und
einer Feder (50), die zwischen dem Lanzettenkern und der inneren Hülse angeordnet ist und dem Lanzettenkern Kraft verleiht, um sich axial entlang des Lanzettenkern-Führungsabschnitts in Richtung des Lanzettenauslasses zu bewegen,
wobei:
aufgrund der axialen Bewegung der inneren Hülse in Richtung des Lanzettenauslasses die innere Hülse relativ zur äußeren Hülse von einer ersten Umfangsposition in eine zweite Umfangsposition rotiert,
in der ersten Umfangsposition die äußere Hülse an dem Lanzettenkern anliegt, um den Lanzettenkern am Lancieren zu hindern, und in der zweiten Umfangsposition der Lanzettenkern geeignet ist, zum Lancieren in den Lanzettenkernführungsabschnitt einzutreten, und
ein erster Vorsprung (28) an der radialen Außenseite eines Zylinderkörpers der inneren Hülse angeordnet ist, ein erster Blockierabschnitt (312) in der äußeren Hülse angeordnet ist, wenn die innere Hülse in der ersten Umfangsposition ist, befindet sich der erste Vorsprung auf einer Seite des ersten Blockierabschnitts in der Umfangsrichtung, und wenn die innere Hülse in der zweiten Umfangsposition ist, befindet sich der erste Vorsprung auf der anderen Seite des ersten Blockierabschnitts in der Umfangsrichtung, und der erste Blockierabschnitt verhindert, dass sich der erste Vorsprung, der sich auf der anderen Seite des ersten Blockierabschnitts befindet, in Umfangsrichtung zu der einen Seite des ersten Blockierabschnitts bewegt.

2. Blutlanzette nach Anspruch 1, wobei:
die äußere Hülse mit einem zweiten Blockierabschnitt (310) und einem dritten Blockierabschnitt (312) versehen ist, der zweite Blockierabschnitt und der dritte Blockierabschnitt in der Umfangsrichtung aufeinanderfolgend angeordnet sind und der dritte Blockierabschnitt ein umlaufender, geneigter Ausdehnungs-Blockierabschnitt ist,
das andere Ende der inneren Hülse mit einem zweiten Vorsprung (24) versehen ist, der radial nach außen vorsteht, und sowohl der zweite Blockierabschnitt als auch der dritte Blockierabschnitt geeignet sind, mit dem zweiten Vorsprung zusammenzuwirken, um zu verhindern, dass sich der zweite Vorsprung in der Richtung vom Lanzettenauslass zur Aufnahmeöffnung bewegt, und
wenn sich die innere Hülse in der ersten Umfangsposition befindet, liegt der zweite Vorsprung gegen den zweiten Blockierabschnitt an, und wenn sich die innere Hülse in der zweiten Umfangsposition befindet, liegt der zweite Vorsprung gegen den dritten Blockierabschnitt an.

3. Blutlanzette nach Anspruch 2, wobei:
der zweite Blockierabschnitt einen Umfangsvorsprung umfasst, der an der Innenwand der äußeren Hülse angeordnet ist, der Umfangsvorsprung radial nach innen vorsteht und der Umfangsvorsprung eine Blockierfläche (310a) aufweist, die geeignet ist, mit dem zweiten Vorsprung zusammenzuwirken, um zu verhindern, dass sich der zweite Vorsprung in der Richtung vom Lanzettenauslass zur Aufnahmeöffnung bewegt.

4. Blutlanzette nach Anspruch 3, wobei:
der dritte Blockierabschnitt eine umlaufende, geneigte Ausdehnungs-Blockierfläche (311) aufweist, eine axiale Ausdehnungs-Blockierfläche (316) zwischen der umlaufenden, geneigten Ausdehnungs-Blockierfläche und der Blockierfläche (310a) angeordnet ist, ein Ende der axialen Ausdehnungs-Blockierfläche mit der umlaufenden, geneigten Ausdehnungs-Blockierfläche verbunden ist und ihr anderes Ende mit der Blockierfläche verbunden ist.

5. Blutlanzette nach Anspruch 2, wobei:
das andere Ende der inneren Hülse mit einem elastischen Arm (26) versehen ist und der Endteil des elastischen Arms mit dem zweiten Vorsprung versehen ist, der von dem elastischen Arm radial nach außen vorsteht.

6. Blutlanzette nach Anspruch 5, wobei:
eine geneigte Zusammenwirkungsführungsfläche (34) in der äußeren Hülse angeordnet ist und der zweite Vorsprung geeignet ist, gegen die geneigte Zusammenwirkungsführungsfläche anzuliegen, um den elastischen Arm anzutreiben, sich radial nach innen zu verformen, wobei der zweite Vorsprung ferner alternativ mit einer zweiten konvexen geneigten Fläche versehen ist, die mit der geneigten Zusammenwirkungsführungsfläche zusammenwirkt.

7. Blutlanzette nach Anspruch 2, wobei:
der zweite Blockierabschnitt eine in der Innenwand der äußeren Hülse ausgebildete Nut oder einen Schlitz oder ein durch die Zylinderwand der äußeren Hülse verlaufendes Durchgangsloch umfasst und die Nut oder der Schlitz oder das Durchgangsloch geeignet ist, mit dem zweiten Vorsprung zusammenzuwirken, um zu verhindern, dass sich der zweite Vorsprung in der Richtung von der Aufnahmeöffnung zum Lanzettenausgang bewegt, oder
der zweite Blockierabschnitt einen geneigten Ausleger umfasst, der radial nach außen elastisch verformbar ist und dessen eines Ende mit der Innenwand der äußeren Hülse verbunden ist, während sich das andere Ende schräg nach innen in Richtung des Lanzettenauslasses erstreckt, der zweite Vorsprung den geneigten Ausleger radial nach außen drängt, während er durch den geneigten Ausleger hindurchgeht, und nachdem der zweite Vorsprung durch den geneigten Ausleger hindurchgegangen ist, sich das andere Ende des geneigten Auslegers radial nach innen bewegt, um zu verhindern, dass sich der zweite Vorsprung in der Richtung von der Aufnahmeöffnung zum Lanzettenauslass bewegt.

8. Blutlanzette nach einem der Ansprüche 1-7, wobei:
eine Positionierungsfläche (37), die an den Lanzettenkern-Führungsabschnitt (36) angrenzt, in der äußeren Hülse angeordnet ist, und der Lanzettenkern eine Anschlagsendfläche (18) aufweist, die an der Positionierungsfläche anliegt,
ein Drehführungsabschnitt (313) in der äußeren Hülse angeordnet ist und ein Ende der inneren Hülse mit einem Drehzusammenwirkungsabschnitt (29) versehen ist, der mit dem Drehführungsabschnitt zusammenwirkt, und
basierend auf der axialen Bewegung der inneren Hülse in Richtung des Lanzettenauslasses der Drehzusammenwirkungsabschnitt (29) mit dem Drehführungsabschnitt (313) zusammenwirkt, um zu bewirken, dass sich die innere Hülse in der Umfangsrichtung dreht, und basierend auf der Drehung die Anschlagsendfläche (18) geeignet ist, die Positionierungsoberfläche zu verlassen, um in den Lanzettenkernführungsabschnitt (36) einzutreten.

9. Blutlanzette nach Anspruch 8, wobei:
die innere Hülse mit einer Führungsnut (21) versehen ist, der Lanzettenkern mit einem Führungselement (15) versehen ist, das mit der Führungsnut zusammenwirkt, das Führungselement in der Führungsnut angeordnet ist, und die Führungsnut geeignet ist, das Führungselement anzutreiben, um den Lanzettenkern anzutreiben, sich entlang der Umfangsrichtung zu drehen, wobei ferner alternativ eine Endfläche des Führungselements (15) die Anschlagsendfläche (18) bildet.

10. Blutlanzette nach Anspruch 9, wobei:
der Drehführungsabschnitt (313) eine umlaufende, geneigte Fläche aufweist,
der Endteil der Führungsnut den Drehzusammenwirkungsabschnitt bildet, der geeignet ist, mit der umlaufenden, geneigten Fläche in Eingriff gebracht zu werden, oder die Außenseite der inneren Hülse mit einem Zusammenwirkungsflansch versehen ist, der radial nach außen vorsteht, und der Zusammenwirkungsflansch den Drehzusammenwirkungsabschnitt bildet, der geeignet ist, mit der umlaufenden, geneigten Fläche in Eingriff gebracht zu werden.

11. Blutlanzette nach Anspruch 10, wobei:
der Drehführungsabschnitt (313) eine geneigte Rippe umfasst, die in der äußeren Hülse angeordnet ist, und die geneigte Rippe radial nach innen ragt und die umlaufende, geneigte Fläche aufweist,
weiter alternativ,
die umlaufende, geneigte Fläche auf der Außenseite des Lanzettenkern-Führungsabschnitts in der radialen Richtung angeordnet ist, und die äußere Hülse ferner mit einer Umfangsebene (314) versehen ist, die in Kontakt mit der umlaufenden, geneigten Fläche steht, und in dem Fall, dass sich der Drehzusammenwirkungsabschnitt von der umlaufenden, geneigten Fläche zu der Umfangsebene auf der Grundlage der Drehung der inneren Hülse bewegt, die Führungsnut und der Lanzettenkern-Führungsabschnitt in der axialen Richtung ausgerichtet sind, um es dem Führungselement zu ermöglichen, sich von der Führungsnut zu dem Lanzettenkern-Führungsabschnitt zu bewegen.

12. Blutlanzette nach Anspruch 8, wobei:
der Drehführungsabschnitt eine geneigte Führungsnut aufweist,
der Drehzusammenwirkungsabschnitt einen Zusammenwirkungsvorsprung umfasst, der von der inneren Hülse radial nach außen vorsteht, und der Zusammenwirkungsvorsprung geeignet ist, sich in der geneigten Führungsnut zu bewegen.

13. Blutlanzette nach einem der Ansprüche 1-7, wobei:
in der axialen Richtung ein Anschlagelement in der inneren Hülse an dem Lanzettenkern anliegt, um eine Anschlagskraft in Richtung des Lanzettenauslasses auszuüben, ein Bauteil in der äußeren Hülse an dem Lanzettenkern anliegt, um eine Anschlagskraft in Richtung der Aufnahmeöffnung auszuüben, und der Lanzettenkern an dem Anschlagelement in der inneren Hülse anliegt und gleichzeitig an der äußeren Hülse anliegt, um zu verhindern, dass sich die innere Hülse in Richtung des Lanzettenauslasses in der axialen Richtung bewegt.

14. Blutlanzette nach Anspruch 13, wobei:
der Lanzettenkern an dem Ende, an dem die Feder vorgesehen ist, mit einer Stützfläche (14) versehen ist, und das Anschlagelement und die Stützfläche geeignet sind, aneinander anzuliegen,
der Lanzettenkern ferner eine Lanzettenkappe (11) umfasst, die auf dem Lanzettenkörper angeordnet ist, die Lanzettenkappe durch die äußere Hülse durch den Lanzettenauslass hindurchgeht, die Lanzettenkappe mit einer Lanzettenkappenaufhängeplattform (12) versehen ist, die geeignet ist, an einer inneren Endfläche (315) eines Endes der äußeren Hülse auf Grund der potentiellen Energie der Feder anzuliegen, und der Lanzettenauslass mit einem Öffnungsdurchgang versehen ist, der es der Lanzettenkappenaufhängeplattform ermöglicht, nur in einer bestimmten Richtung hindurchzugehen, und
der Anschlag zwischen der Stützfläche (14) und dem Anschlagelement (23) und der Anschlag zwischen der Lanzettenkappen-Aufhängeplattform (12) und der inneren Endfläche (315) der äußeren Hülse gemeinsam verhindern, dass sich die innere Hülse in axialer Richtung in das Innere der äußeren Hülse bewegt.

15. Blutlanzette nach Anspruch 14, wobei:
eine Positionierungsfläche (37), die an den Lanzettenkern-Führungsabschnitt (36) angrenzt, in der äußeren Hülse angeordnet ist, und der Lanzettenkern eine Anschlagsendfläche (18) aufweist, die an der Positionierungsfläche anliegt, und
für den Fall, dass der Lanzettenkern an dem Anschlagelement in der inneren Hülse und der Lanzettenkern an der äußere Hülse anliegt, ein vorbestimmter Abstand zwischen der Anschlagsendfläche und der Positionierungsfläche in axialer Richtung besteht.

## Revendications

1. Lancette pour prise d'échantillons sanguins comprenant :
un manchon externe (30) prévu avec une sortie de lancette (31) au niveau d'une extrémité et un orifice de logement au niveau de l'autre extrémité, une partie de guidage d'âme de lancette (36) étant agencée dans le manchon externe ;
un manchon interne (20), une extrémité du manchon interne pénétrant dans le manchon externe depuis l'orifice de logement et l'autre extrémité du manchon interne étant située à l'extérieur de l'orifice de logement ;
une âme de lancette (10) prévue avec un corps de lancette, l'âme de lancette étant agencée au niveau d'une extrémité du manchon interne afin de tourner selon la direction circonférentielle avec le manchon interne ; et
un ressort (50), qui est agencé entre l'âme de lancette et le manchon interne, et fournit la puissance pour que l'âme de lancette se déplace axialement le long de la partie de guidage d'âme de lancette vers la sortie de lancette,
dans laquelle :
sur la base du mouvement axial du manchon interne dans la direction vers la sortie de lancette, le manchon interne tourne par rapport au manchon externe d'une première position circonférentielle à une seconde position circonférentielle ;
dans la première position circonférentielle, le manchon externe vient en butée contre l'âme de lancette pour empêcher le lancement de l'âme de lancette, et dans la seconde position circonférentielle, l'âme de lancette est appropriée pour pénétrer dans la partie de guidage d'âme de lancette pour le lancement ; et
une première saillie (28) est agencée sur le côté externe radial d'un corps cylindrique du manchon interne, une première partie de blocage (312) est agencée dans le manchon externe, lorsque le manchon interne est dans la première position circonférentielle, la première saillie est positionnée d'un côté de la première partie de blocage dans la direction circonférentielle ; et lorsque le manchon interne est dans la seconde position circonférentielle, la première saillie est positionnée de l'autre côté de la première partie de blocage dans la direction circonférentielle, et la première partie de blocage empêche la première saillie positionnée de l'autre côté de la première partie de blocage de se déplacer de manière circonférentielle vers ledit un côté de la première partie de blocage.

2. Lancette pour prise d'échantillons sanguins selon la revendication 1, dans laquelle :
le manchon externe est prévu avec une deuxième partie de blocage (310) et une troisième partie de blocage (312), la deuxième partie de blocage et la troisième partie de blocage sont agencées en séquence selon la direction circonférentielle, et la troisième partie de blocage est une partie de blocage à extension circonférentielle inclinée ;
l'autre extrémité du manchon interne est prévue avec une seconde saillie (24) faisant saillie radialement vers l'extérieur, et à la fois la deuxième partie de blocage et la troisième partie de blocage sont appropriées pour coopérer avec la seconde saillie afin d'empêcher la seconde saillie de se déplacer dans la direction allant de la sortie de lancette à l'orifice de logement ; et
lorsque le manchon interne est dans la première position circonférentielle, la seconde saillie vient en butée contre la deuxième partie de blocage, et lorsque le manchon interne est dans la seconde position circonférentielle, la seconde saillie vient en butée contre la troisième partie de blocage.

3. Lancette pour prise d'échantillons sanguins selon la revendication 2, dans laquelle :
la deuxième partie de blocage comprend une saillie circonférentielle agencée sur la paroi interne du manchon externe, la saillie circonférentielle fait saillie radialement vers l'intérieur, et la saillie circonférentielle a une surface de blocage (310a) qui est appropriée pour coopérer avec la seconde saillie afin d'empêcher la seconde saillie de se déplacer dans la direction allant de la sortie de lancette à l'orifice de logement.

4. Lancette pour prise d'échantillons sanguins selon la revendication 3, dans laquelle :
la troisième partie de blocage comprend une surface de blocage à extension circonférentielle inclinée (311), une surface de blocage à extension axiale (316) est agencée entre la surface de blocage à extension circonférentielle inclinée et la surface de blocage (310a), une extrémité de la surface de blocage à extension axiale est raccordée à la surface de blocage à extension circonférentielle inclinée, et son autre extrémité est raccordée à la surface de blocage.

5. Lancette pour prise d'échantillons sanguins selon la revendication 2, dans laquelle :
l'autre extrémité du manchon interne est prévue avec un bras élastique (26), et la partie d'extrémité du bras élastique est prévue avec la seconde saillie faisant saillie radialement vers l'extérieur à partir du bras élastique.

6. Lancette pour prise d'échantillons sanguins selon la revendication 5, dans laquelle :
une surface inclinée de guidage par coopération (34) est agencée dans le manchon externe, et la seconde saillie est appropriée pour venir en butée contre la surface inclinée de guidage par coopération afin d'entraîner le bras élastique pour qu'il se déforme radialement vers l'intérieur, en outre en variante, la seconde saillie est prévue avec une seconde surface inclinée convexe qui coopère avec la surface inclinée de guidage par coopération.

7. Lancette pour prise d'échantillons sanguins selon la revendication 2, dans laquelle :
la seconde partie de blocage comprend une rainure ou une fente formée dans la paroi interne du manchon externe ou un trou débouchant passant par la paroi cylindrique du manchon externe, et la rainure ou la fente ou le trou débouchant est approprié(e) pour coopérer avec la seconde saillie pour empêcher la seconde saillie de se déplacer dans la direction allant de l'orifice de logement à la sortie de lancette ; ou bien
la seconde partie de blocage comprend un porte-à-faux incliné qui est élastiquement déformable radialement vers l'extérieur et dont une extrémité est raccordée à la paroi interne du manchon externe alors que son autre extrémité s'étend de manière oblique vers l'intérieur vers la sortie de lancette, la seconde saillie déforme le porte-à-faux incliné radialement vers l'extérieur pendant le passage par le porte-à-faux incliné et après que la seconde saillie est passée par le porte-à-faux incliné, l'autre extrémité du porte-à-faux incliné se déplace radialement vers l'intérieur pour empêcher la seconde saillie de se déplacer dans la direction allant de l'orifice de logement à la sortie de lancette.

8. Lancette pour prise d'échantillons sanguins selon l'une quelconque des revendications 1 à 7, dans laquelle :
une surface de positionnement (37) adjacente à la partie de guidage d'âme de lancette (36) est agencée dans le manchon externe, et l'âme de lancette a une face d'extrémité en butée (18) qui vient en butée contre la surface de positionnement ;
une partie de guidage de rotation (313) est agencée dans le manchon externe, et une extrémité du manchon interne est prévue avec une partie de coopération de rotation (29) qui coopère avec la partie de guidage de rotation ; et
sur la base du mouvement axial du manchon interne dans la direction vers la sortie de lancette, la partie de coopération de rotation (29) coopère avec la partie de guidage de rotation (313) pour amener le manchon interne à tourner dans la direction circonférentielle, et sur la base de la rotation, la face d'extrémité en butée (18) est appropriée pour quitter la surface de positionnement afin d'entrer dans la partie de guidage d'âme de lancette (36).

9. Lancette pour prise d'échantillons sanguins selon la revendication 8, dans laquelle :
le manchon interne est prévu avec une rainure de guidage (21), l'âme de lancette est prévue avec un élément de guidage (15) qui coopère avec la rainure de guidage, l'élément de guidage est agencé dans la rainure de guidage, et la rainure de guidage est appropriée pour entraîner l'élément de guidage afin d'entraîner l'âme de lancette à tourner selon la direction circonférentielle, en outre en variante, une face d'extrémité de l'élément de guidage (15) constitue la surface d'extrémité en butée (18).

10. Lancette pour prise d'échantillons sanguins selon la revendication 9, dans laquelle :
la partie de guidage de rotation (313) comprend une surface circonférentielle inclinée ;
la partie d'extrémité de la rainure de guidage constitue la partie de coopération de rotation afin d'être appropriée pour être mise en prise avec la surface circonférentielle inclinée ; ou le côté externe du manchon interne est prévu avec une bride de coopération faisant saillie radialement vers l'extérieur, et la bride de coopération constitue la partie de coopération de rotation afin d'être appropriée pour être mise en prise avec la surface circonférentielle inclinée.

11. Lancette pour prise d'échantillons sanguins selon la revendication 10, dans laquelle :
la partie de guidage de rotation (313) comprend une nervure inclinée agencée dans le manchon externe, et la nervure inclinée fait saillie radialement vers l'intérieur et a la surface circonférentielle inclinée,
en outre en variante,
la surface circonférentielle inclinée est positionnée sur le côté externe de la partie de guidage d'âme de lancette dans la direction radiale ; et le manchon externe est en outre prévu avec un plan circonférentiel (314) qui est en contact avec la surface circonférentielle inclinée, et dans le cas dans lequel la partie de coopération de rotation se déplace de la surface circonférentielle inclinée au plan circonférentiel sur la base de la rotation du manchon interne, la rainure de guidage et la partie de guidage d'âme de lancette sont alignées dans la direction axiale pour permettre à l'élément de guidage de se déplacer de la rainure de guidage à la partie de guidage d'âme de lancette.

12. Lancette pour prise d'échantillons sanguins selon la revendication 8, dans laquelle :
la partie de guidage de rotation comprend une rainure de guidage inclinée ;
la partie de coopération de rotation comprend une saillie de coopération qui fait saillie radialement vers l'extérieur à partir du manchon interne, et la saillie de coopération est appropriée pour se déplacer dans la rainure de guidage inclinée.

13. Lancette pour prise d'échantillons sanguins selon l'une quelconque des revendications 1 à 7, dans laquelle :
dans la direction axiale, un élément de butée dans le manchon interne vient en butée contre l'âme de lancette pour appliquer une force de butée vers la direction de la sortie de lancette, un composant dans le manchon externe vient en butée contre l'âme de lancette afin d'appliquer une force de butée vers l'orifice de logement, et l'âme de lancette vient en butée contre l'élément de butée dans le manchon interne et vient en butée contre le manchon externe en même temps pour empêcher le déplacement du manchon interne vers la sortie de lancette dans la direction axiale.

14. Lancette pour prise d'échantillons sanguins selon la revendication 13, dans laquelle :
l'âme de lancette est prévue avec une surface de support (14) à l'extrémité où le ressort est prévu, et l'élément de butée et la surface de support sont appropriés pour venir en butée l'un contre l'autre ;
l'âme de lancette comprend en outre un capuchon de lancette (11) agencé sur le corps de lancette, le capuchon de lancette passe par le manchon externe par la sortie de lancette, le capuchon de lancette est prévu avec une plateforme de suspension de capuchon de lancette (12) qui est appropriée pour venir en butée contre une face d'extrémité interne (315) d'une extrémité du manchon externe sur la base de l'énergie potentielle du ressort, et la sortie de lancette est prévue avec un passage d'ouverture qui permet à la plateforme de suspension de capuchon de lancette de traverser uniquement dans une direction spécifique ; et
la butée entre la surface de support (14) et l'élément de butée (23) et la butée entre la plateforme de suspension de capuchon de lancette (12) et la surface d'extrémité interne (315) du manchon externe empêchent conjointement le manchon interne de se déplacer dans la direction axiale vers l'intérieur du manchon externe.

15. Lancette pour prise d'échantillons sanguins selon la revendication 14, dans laquelle :
une surface de positionnement (37) adjacente à la partie de guidage d'âme de lancette (36) est agencée dans le manchon externe, et l'âme de lancette a une face d'extrémité en butée (18) qui vient en butée contre la surface de positionnement ; et
dans le cas où l'âme de lancette vient en butée contre l'élément de butée dans le manchon interne et où l'âme de lancette vient en butée contre le manchon externe, il y a une distance prédéterminée entre la surface d'extrémité de butée et la surface de positionnement dans la direction axiale.
